Europäisches Patentamt

European Patent Office

Office européen des brevets

(11) Veröffentlichungsnummer : **0 070 956**
**B1**

## EUROPÄISCHE PATENTSCHRIFT

(45) Veröffentlichungstag der Patentschrift :
30.12.86

(51) Int. Cl.⁴ : **A 61 F  5/47**

(21) Anmeldenummer : **82100394.4**

(22) Anmeldetag : **21.01.82**

(54) Intrauterine empfängnisverhütende Vorrichtung.

(43) Veröffentlichungstag der Anmeldung :
09.02.83 Patentblatt 83/06

(45) Bekanntmachung des Hinweises auf die Patenterteilung : 30.12.86 Patentblatt 86/52

(84) Benannte Vertragsstaaten :
DE FR GB NL

(56) Entgegenhaltungen :
FR-A- 2 085 578
US-A- 3 407 806
US-A- 3 789 838
US-A- 3 937 217

(73) Patentinhaber : **Internationales Forschungsinstitut für Reproduktionsmedizin und - biologie nachfolgend IRIR (International Research Institute for Reproduction Kaiser-Wilhelm-Ring 22 D-4000 Düsseldorf (DE)**

(72) Erfinder : **Kurz, Karl Heinz, Dr.med. Kaiser Wilhelm Ring 22 D-4000 Düsseldorf (DE)**
Erfinder : **Gutierrez, Xavier, Dipl.-Ing. Blumenstrasse 5 D-4005 Meerbusch (DE)**

(74) Vertreter : **Tackenberg, Karl, Dipl.-Ing. Birkenweiher 15 D-5650 Solingen (DE)**

## Beschreibung

Die Erfindung betrifft eine intrauterine, empfängnisverhütende, aus einem Schaft und zwei von einem Ende des Schafts ausgehenden Armen bestehende Vorrichtung aus elastischem Material, wobei der Schaft aus zwei am anderen Ende des Schafts fest miteinander verbundenen Stielen besteht.

Eine derartige Vorrichtung ist bekannt (FR-A-2 085 578). Die bekannte Vorrichtung hat die Form eines Ypsilon. Bei Druck auf die Arme der Vorrichtung, hervorgerufen durch Kontraktion des Uterus und dadurch erfolgende Verkleinerung der Gebärmutterhöhle, besteht die Gefahr, daß die Arme in die Seitenwände des Uterus eingedrückt werden, was zu Verletzungen von Schleimhaut und Muskulatur führt und Unverträglichkeit der Vorrichtung zur Folge hat.

Der Erfindung liegt die Aufgabe zugrunde, eine Vorrichtung der eingangs genannten Gattung so auszubilden, daß bei Druck auf die Arme der Vorrichtung eine Verletzung der Seitenwände des Uterus vermieden ist.

Die Lösung der Aufgabe besteht erfindungsgemäß darin, daß die Stiele im unbelasteten Zustand parallel zueinander verlaufen und daß die Vorrichtung die Form eines T aufweist, wobei die Arme des T zum Schaft hin gekrümmt sind und sich im unbelasteten Zustand kreuzen, so daß sich die Stiele unter einer über die Arme eingeleiteten Biegebeanspruchung voneinander weg krümmen und bei einer Kontraktion des Uterus an die seitlichen Wände des Uterus sanft anlegen.

Die bei Druck auf die Arme der Vorrichtung voneinander weg krümmenden Stiele legen sich mit den Krümmungen zwangsweise und sanft an die Seitenwände des Uterus an. Hierdurch ist eine Verletzung der Seitenwände des Uterus vermieden.

Die Erfindung ist nachstehend anhand der Zeichnung näher erläutert. Es zeigt :

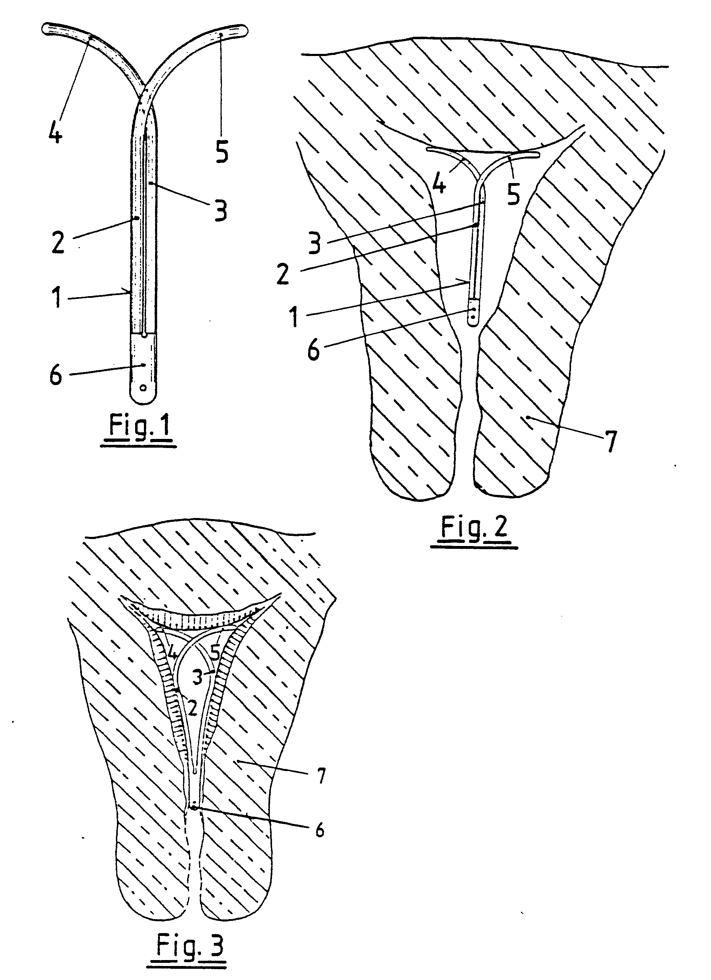

Figur 1 die Vorrichtung in Ansicht,
Figur 2 die Vorrichtung in einem Uterus vor dessen Kontraktion und
Figur 3 bei Kontraktion des Uterus.

Die aus elastischem Material bestehende Vorrichtung weist die Form eines T auf. Sie besteht aus einem Schaft 1, der sich aus zwei Stielen 2, 3 zusammensetzt, die im unbelasteten Zustand parallel zueinander verlaufen. Die Stiele 2, 3 weisen von einem Ende des Schaftes 1 ausgehende Arme 4, 5 auf, die sich in unbelastetem Zustand kreuzen und zum Schaft 1 hin gekrümmt sind. Sie sind am anderen Ende des Schaftes 1 bei 6 fest miteinander verbunden. Mit 7 ist ein Uterus bezeichnet.

Bei Kontraktion des Uterus 7 und dabei auf die Arme 4, 5 ausgeübtem Druck krümmen die Stiele 2, 3 voneinander weg und legen sich sanft an die Seitenwände des Uterus 7 an.

## Patentanspruch

Intrauterine, empfängnisverhütende, aus einem Schaft (1) und zwei von einem Ende des Schafts ausgehenden Armen (4, 5) bestehende Vorrichtung aus elastischem Material, wobei der Schaft (1) aus zwei am anderen Ende des Schafts (1) fest miteinander verbundenen Stielen (2, 3) besteht, dadurch gekennzeichnet, daß die Stiele (2, 3) im unbelasteten Zustand parallel zueinander verlaufen und daß die Vorrichtung die Form eines T aufweist, wobei die Arme (4, 5) des T zum Schaft (1) hin gekrümmt sind und sich im unbelasteten Zustand kreuzen, so daß sich die Stiele (2, 3) unter einer über die Arme (4, 5) eingeleiteten Biegebeanspruchung voneinander weg krümmen und bei einer Kontraktion des Uterus an die seitlichen Wände des Uterus sanft anlegen.

## Claim

Intra-uterine contraceptive device of resilient material, comprising a shaft (1) and two arms (4, 5) starting from one end of the shaft, the shaft (1) consisting of two posts (2, 3) which are firmly connected to one another at the other end of the shaft (1), characterised in that the posts (2, 3) extend parallel to one another in the unloaded condition and that the device has the shape of a T, the arms (4, 5) of the T being curved towards the shaft (1) and cross each other in the unloaded condition, so that the posts (2, 3) under a flexural stress originating from the arms (4, 5) bend away from one another and in the case of a contraction of the uterus smoothly engage the lateral walls of the uterus.

## Revendication

Dispositif contraceptif intra-utérin en matière plastique, composé d'un fût (1) et de deux bras (4, 5) partant d'une extrémité du fût qui, lui, se compose de deux tiges (2, 3) solidement unies l'une à l'autre, à l'autre extrémité du fût (1), dispositif caractérisé par ce que les tiges (2, 3) sont parallèles l'une par rapport à l'autre quand elles ne sont pas soumises à un effort et que le dispositif se présente sous la forme d'un T dont les bras (4, 5) sont cintrés vers le fût (1) et se croisent quand ils ne sont pas soumis à un effort de sorte que les tiges (2, 3) s'éloignent l'une de l'autre en se courbant sous l'effort de flexion amorcé par l'intermédiaire des bras (4, 5) et s'appliquent doucement aux parois latérales de l'utérus quand celui-ci se contracte.

Fig. 1

Fig. 2

Fig. 3